# EUROPEAN PATENT APPLICATION

(11) **EP 2 525 208 A1**
(43) Date of publication of application: **21.11.2012**
(21) Application number: 11731705.7
(22) Date of filing: 11.01.2011
(51) Int. Cl.: G01N 15/14, G01N 21/05, G01N 21/85

(54) **DEVICE AND SYSTEM FOR COUNTING AND ANALYSING PARTICLES AND USE OF SAID SYSTEM**

(30) Priority: 11.01.2010 ES 201030015
(71) Applicant: Consejo Superior De Investigaciones Científicas (CSIC), 28006 Madrid (ES)
(72) Inventor: LLOBERA ADÁN, Andreu, E-08193 Cerdanyola del Vallès (Barcelona) (ES)
(74) Representative: Pons Ariño, Angel
(86) International application number: PCT/ES2011/070011
(87) International publication number: WO 2011/083200

(57) **Abstract**

The invention relates to an MIR-type device for analysing and taking measurements in relation to particles using optical means, particularly suprananometric particles located in a suspension injected into the device. The invention also relates to a particle analysis system that uses the device which is connected to a light source and a read unit.

## Description

### OBJECT OF THE INVENTION

The present invention relates to the technical field of detection, analysis and counting of particles using a disposable optofluidic system whereto a light source and reading unit is coupled.

### BACKGROUND OF THE INVENTION

Various solutions to the problem of detecting and analysing very small particles, specifically of suprananometric size, are known to exist in the current state of the art.

For example, a Neubauer cell is commonly used to count particles (mainly cells). However, it lacks precision due to user subjectivity. Likewise, a single measurement is insufficient to confirm the particle count, as it is possible to have an uneven distribution within the cell that gives rise to an incorrect number. Therefore, random repetition is required in order to offset said problem.

Flow cytometry is a serial counting system (consecutive measurement) which allows more exact determination than a Neubauer cell. However, it requires lengthy measurement times, as well as complex and expensive equipment. Additionally, neither of the two allows simultaneous analysis of the particles.

If a fluid with two types of suprananometric particles is assumed: absorbent and non-absorbent at certain wavelengths. The spectrophotometers could determine the optical density of said particles only if its wavelength coincides with the particle absorption bands, otherwise they are difficult to detect. A LUCAS system detects both types of particles but does not allow discernment therebetween (when their dimensions are comparable) nor determination of their properties. Flow cytometers do allow differentiation thereof, but the measurement is serial and consecutive, requiring long total interrogation times of a defined sample volume.

### DESCRIPTION OF THE INVENTION

The system object of the invention allows both analysis and detection of particles injected in the interior thereof, such as cells, regardless of the possible labelling thereof, as it has various analysis methods which allows optimisation of the measurement based on the properties of the particles to be measured. In addition, the system presented allows continuous analysis, whereupon differentiations or variations in the suprananometric particles can be determined. Likewise, the analysis methods allow both uniparametric measurements (a single magnitude) and multiparametric measurements (different magnitudes) in analysis times of approximately 30 milliseconds.

The main object of the invention is the use of a system for analysing and detecting particles having a multiple internal reflection (MIR) device (hereinafter "MIR") for the quasi-simultaneous multiparametric detection and measurement of particles, said device also being disposable or reusable. Said system consists of a fluid cell wherein fluids can be introduced by means of fluid inlets in order to be analysed by means of an analysis wherein a light source such as optical fibre is coupled to said cell, allowing the optical properties of the injected fluid and, by extension, of the particles dispersed therein to be determined.

One of the differentiating characteristics of the system object of the invention lies in the use of a wide spectrum source for injection and a spectral measurement system for collection to enable multiparametric detection in a single measurement. These two properties solve the problems inherent to current spectrophotometers (which measure optical density at a fixed wavelength), flow cytometers (which perform serial measurements) and to configurations based on count by image recognition (LUCAS in English, which allow counting but not analysis thereof).

The system object of the invention consists of the use of a multiple internal reflection device for the detection, analysis and/or count of particles suspended in a liquid. Said MIR device is defined in a chip and comprises air mirrors defined by hollow structures in the form of curved slots near the analysis zone, corresponding to the so-called interrogation zone, which is the zone where the light interacts with the liquid to be analysed by the system. The air mirrors propagate the light in a zig-zag path, allowing elongation of the optical path and keeping system dimensions within reasonable margins. The aforementioned device comprises several additional elements, such as self-alignment channels or automatic alignment, the aforementioned air mirrors and micro-lenses for rectifying the light beam, preferably housed in said self-alignment grooves, the device object of the invention is defined by a single photolithography mask, which can be applied to low-cost materials such as polymeric materials, for example PDMS.

Particle analysis, detection or counting systems generally function under one of the following regimes: LS ("large scattering" dispersion with angles between 15° and 150°), LS+ABS ("scattering" dispersion + absorption) and ABS (absorption). On the contrary, the differentiating factor of this patent is that the system object of the invention can function simultaneously under the three aforementioned regimes.

As opposed to what occurs in the case of cytometers, where the suprananometric particles, normally cells, are counted sequentially, the system object of the invention performs a single measurement for 30 ms in the entire area; in the event that the cells are not marked or do not have absorption bands, a dispersion spectrum is obtained; if the cells are marked a superimposed absorbance band is observed. In both regimes, LS and ABS+LS, the number of particles present can be counted.

The system can obtain the spectrum relative only to absorption (ABS) by subtraction of the two results mentioned in the preceding paragraph. Likewise, the system not only allows counting of a cell population, but also allows the establishment of a marked/unmarked cell rate using two or more different markers. Additionally, if a differentiation of said particles occurs (such as that due to cellular growth or change in the properties thereof), it could also be detected by the system proposed.

An additional factor of the system object of the invention which no other current system has is its portability. The system object of the invention may be manufactured using both microelectronic technology and polymer technology, as described previously. Once the geometry has been defined and once the refraction indices of the materials to be used are known, said systems can be manufactured with minimum complexity.

### DESCRIPTION OF THE DRAWINGS

In order to complete the description being made and with the object of helping to better understand the characteristics of the invention, in accordance with a preferred example of practical embodiment thereof, accompanying said description as an integral part thereof is a set of drawings wherein the following has been represented in an illustrative and non-limiting manner:
Figure 1 shows a three-dimensional view of the system object of the invention.
Figure 2 shows a schematic view of the system object of the invention.

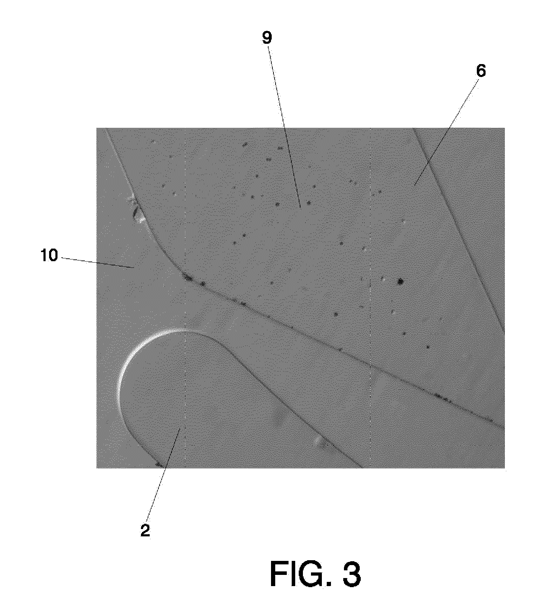

Figure 3 shows a detailed view of the interrogation system object of the invention.

### PREFERRED EMBODIMENT OF THE INVENTION

In light of the aforementioned figures, a preferred embodiment of the device (1) object of the invention is described below.

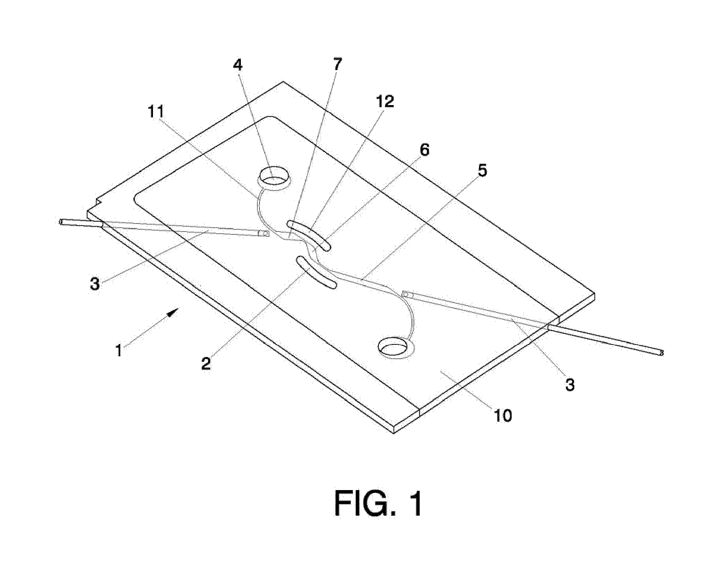

The embodiment was carried out using a device (1) manufactured using lithographic techniques over a transparent polymeric body (10) wherein, as can be observed in figure 1, self-alignment grooves (3) have been defined which will house emitting/receiving optical fibres and micro-lenses (8, 13) disposed at the end of said self-alignment grooves (3). In turn, air mirrors (2, 12) manufactured from hollow curved slots are defined, fluid inlets (4) among which a groove (11) is defined, the central path of which comprises several sections (5, 6, 7) where the air mirrors (2) are defined parallel to the shortest sides of a second section (6) of the groove (11).

In a preferred embodiment, a broadband light source is used as a light source, such as that manufactured by Ocean Optics HL-2000, coupled to a 230 µm in diameter optical fibre with multi-mode reception. Likewise, a reading unit, a spectrometer, whereto an emitting optical fibre identical to that mentioned earlier which transmits the signal sent to the spectrometer is coupled, such as that manufactured by Ocean Optics HR4000 with a spectral resolution of 0.2 nm. An analysis time of 30 ms allows obtainment of the spectrums of the injected particles. The experiment is conducted in a room with a controlled temperature.

First of all, the fluid inlets (4) and the groove (11) are filled with PBS solution, said groove comprising narrow curved paths in the areas adjacent to said fluid inlets (4) and a central zig-zag path defined by the succession of the rhomboid sections (5, 6, 7); once the device is filled (1), a first measurement is performed by emitting a light beam using the light source which crosses a micro-lens (8) disposed in the self-alignment groove (3) which houses the emitting optical fibre and the measurement is made using the emission reading unit to establish a reference measurement under these conditions, which will be used as a reference measurement for the rest of the measurements.

In order to perform measurements in LS and LS+ABS, dissolved concentrations of live cells (unmarked) or dead cells (marked) are injected into the device (1) in variable concentrations of between 50 and 2,000 kcells/ml. The marker used for the dead cells is trypan blue, as it can be used at ambient temperature with an absorption peak located at a wavelength of 581 nm. For each concentration of cells, ten consecutive scans are performed. Once the measurements with the highest concentration are performed, PBS is injected once again to determine possible fluctuations in the reference signal.

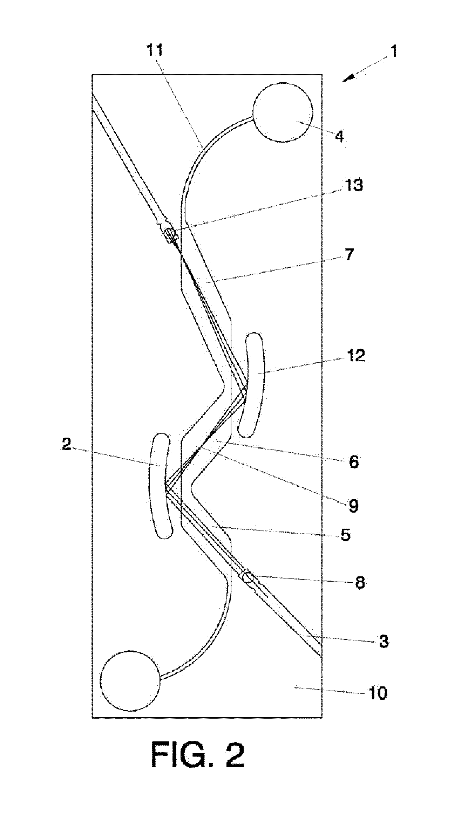

The measurements are performed by introducing optical fibre, one being for emitting, connected to the light source, and another for receiving, connected to the reading unit, in the self-alignment grooves (3) where the emitting optical fibre connected to the light source emits a light beam that penetrates a first micro-lens (8), which is located at the end of a self-alignment groove (3) that houses the emitting optical fibre, and then enters a first rhomboid-shaped section (5) of the groove (11), penetrating the fluid found in said first section (5), which contains the previously injected cells, whereupon the light beam emitted penetrates part of the body (10) until reflected by the action of a first air mirror (2) located in parallel to the first section (5), the centre of curvature of which is disposed in the direction of the longitudinal axis of the alignment groove (3) that houses the emitting optical fibre. The light beam reflected on the air mirror (2) penetrates a second section (6) with a rhomboid-shaped groove (11), wherein the reflected light beam defines an interrogation zone (9), corresponding to the zone where the light beams cross each other's path and where the analysis that can be observed in detail in figure 2 is carried out, before being reflected again onto a second air mirror (12) to penetrate a third section (7), also rhomboid-shaped, until reaching a second micro-lens (13) located in the alignment groove (3) which houses the receiving optical fibre connected to the spectrometer. Said spectrometer receives the light beam that penetrates the fluid and has been reflected by the air mirrors (2, 12).

## Claims

1. Particle counting and analysis device (1), **characterised in that** it consists of a transparent body (10) comprising:
- fluid inlets (4) defined at the corners thereof, wherebetween a groove (11) runs which comprises two first curved sections with parallel walls connected to said fluid inlets (4) having a smaller section than three adjacent rhomboid-shaped sections (5, 6, 7) defined between said first sections,
- air mirrors (2, 12) defined by hollow structures in the form of curved slots disposed on either side of a second section (6) of the groove (11) which define an interrogation zone (9) in said second section (6), and
- self-alignment grooves (3), defined in the interior thereof, in charge of housing optical fibres.

2. Device (1), according to claim 1, **characterised in that** it additionally comprises micro-lenses (8, 13) respectively located at the ends of the self-alignment grooves (3).

3. Device (1), according to claim 2, **characterised in that** the micro-lenses (8, 13) are cylindrical.

4. Device (1), according to any of the preceding claims, **characterised in that** the body (10) is made of a material selected from among the following: a polymeric material, a ceramic material, a semi-conductor material, an insulating material and a conducting material.

5. Device (1), according to claim 4, **characterised in that** the polymeric material is PDMS.

6. Device (1), according to claim 5, **characterised in that** the PDMS is functionalised.

7. Particle counting and analysis system, **characterised in that** it comprises the device (1) described in any of the preceding claims and **in that** it also comprises optical fibres in the interior of the self-alignment grooves (3), respectively, connected to a light-emitting source and to a reading source.

8. System, according to claim 7, where the light source is a broadband light source.

9. System, according to claim 7 or 8, where the reading unit is a spectrometer.

10. System, according to any one of claims 7 to 9, **characterised in that** it additionally comprises fluid injection means connected to the device (1).

11. Use of the system described in claims 7 to 10 for detecting particles in suspension in fluids.

12. Use of the system described in claims 7 to 10 for counting particles in suspension in fluids.

13. Use of the system described in claims 7 to 10 for analysing particles in suspension in fluids.

14. Use of the system described in claims 7 to 10 for analysing particles susceptible to temporary differentiation or alteration in number, morphology or variation of their optical properties.

15. Use of the system, according to any one of claims 11 to 13, **characterised in that** the particles are not marked.
